# EUROPEAN PATENT SPECIFICATION

(11) **EP 1 413 434 B1**
(45) Date of publication and mention of the grant of the patent: **19.12.2007**
(21) Application number: 03256451.0
(22) Date of filing: 13.10.2003
(51) Int. Cl.: B41J 2/04, A61J 3/07, A61K 9/48

(54) **Pharmaceutical dosage form and method of making**
Arzneiform und Verfahren zu ihrer Herstellung
Forme pharmaceutique et procédé de préparation

(30) Priority: 24.10.2002 US 280692
(43) Date of publication of application: 28.04.2004
(73) Proprietor: Hewlett-Packard Development Company, L.P., Houston, TX 77070 (US)
(72) Inventor: Dunfield, John Stephen, Corvallis OR 97330 (US); Ayres, James W., Corvallis OR 97330 (US)
(74) Representative: Jackson, Richard Eric

(56) References cited:
- EP-A- 0 369 445
- WO-A-03/005950
- US-A- 4 322 449
- US-A- 4 349 529
- US-A1- 2001 016 177

## Description

### BACKGROUND

### Description of the Art

Oral administration of pharmaceuticals is one of the most widely used methods to provide effective therapy for a variety of illnesses. Many dry medications are typically administered orally to a person in a dosage form such as tablets or capsules, while still others are in liquid form. The release of orally administered medications may occur in the oral cavity such as for buccal or sublingual administration, or may occur in the gastrointestinal tract after the oral dosage form is swallowed. There are, for example, tablets that release drug in the stomach, enteric coated tablets that release the medication in the intestinal tract of the patient, and controlled release dosage forms that release drug in both the stomach and the intestines. Further, many individuals suffer from chronic health problems that require the regular administration of medicaments. Diseases such as diabetes, allergies, epilepsy, heart problems, AIDS, and even cancers require the regular delivery of precise doses of medicaments if patients are to survive over long periods of time.

Most pharmaceuticals involve dosage units in the microgram to milligram range of the purified active ingredient or ingredients. Thus, many pharmaceutical doses in tablet or liquid form are made in formulations of a predetermined quantity of pharmaceutical units in each dose. Such pharmaceutical doses are frequently available in fixed different strengths, such as 50 mg, 100 mg, etc.

Unfortunately, such conventional oral dosage forms suffer from a number of disadvantages. Typically, to effectively handle and dispense small doses a considerable amount of adjuvant material must be added in order that the final dosage form is of a manageable size. Thus, typical methods for manufacturing include the mixing of the pure drug with various other substances commonly referred to as excipients or diluents that are therapeutically inert and acceptable by regulatory bodies, such as the Federal Drug Administration (FDA). Drugs that have a low solubility or a slow rate of dissolution may be micronized. Micronization decreases the particle size and increases the surface area, which helps increase the rate of drug dissolution. However, micronization is typically a harsh process that can destroy some chemical structures and usually requires that the micronized drug be distributed on the surface of a carrier to minimize agglomeration, however, immediate and long-term agglomeration is still a problem with micronized drugs. Excipients may also protect the drug from deterioration by oxidation, humidity, and light. Palatability can be improved through the addition of flavorants, and identification can be improved by the use of colorants. This mixing process often requires the use of sophisticated, complex expensive machinery. Certain excipients may be needed to improve the flowability of the drug and diluents through the mixing machinery and tablet compression equipment.

These therapeutically inactive or inert materials also have the disadvantage that each such material must be evaluated before use in terms of potential incompatibilities with the medicaments present. For example, some of these materials, such as lubricants or binders, may present problems concerning the bioavailability of the active ingredient. Further, the certification of new drugs is a lengthy and costly process involving animal studies followed by chemical trials to establish both the efficacy and safety of the new drug. Because a pharmaceutical's characteristics may be affected by changes in manufacturing and/or packaging, the approval process limits the approval to a particular manufacturing and packaging process.

Drugs with a narrow therapeutic range must also be precisely dosed. If the drug concentration in the patient falls below the range, the desired effect will not occur. However, if the drug concentration in the patient is above the range then the risk of toxic effects increases. Clinicians assume the dose units manufactured are uniform and that generic equivalents have equal bioavailability. The many FDA formulation rejections and recalls for pharmaceuticals that have too high or low of a drug amount; however, are evidence that accuracy and precision are still challenges for pharmaceutical manufacturing.

EP 0 369 445 discloses a solid encapsulated medicament. WO 03/005950, which comprises part of the state of the art under A. 54(3) EPC, discloses a fluid dispenser which dispenses fluid drops of pharmaceutical component onto a tablet.

If these problems persist, many new and potentially life saving beneficial drugs will either be impractical or have limited effectiveness in the dosage forms currently available.

The present invention provides a method of making a pharmaceutical dosage form, a pharmaceutical dosage form, and a bioactive fluid dispensing system as recited in the claims.

### BRIEF DESCRIPTION OF THE DRAWINGS

Fig. 1 a is a perspective view of a fluid ejector head according to an embodiment of the present invention;

Fig. 1b is a perspective view of a fluid ejector head according to an alternate embodiment of the present invention;

Fig. 2a is an isometric cross-sectional view of a fluid ejector body according to an alternate embodiment of the present invention;

Fig. 2b is a perspective view of a portion of the fluid ejector body shown in Fig. 2a according to an embodiment of the present invention;

Fig. 3 is a cross-sectional view of a fluid ejector body according to an alternate embodiment of the present invention;

Fig. 4 is a perspective view of a fluid dispensing system according to an embodiment of the present invention;

Fig. 5a is a perspective view of an exemplary embodiment of a dosage form according to the present invention;

Fig. 5b is a cross-sectional view of the dosage form shown in Fig. 5a according to an embodiment of the present invention;

Fig. 5c is a cross-sectional view of a magnified portion of the dosage form shown in Fig. 5b according to an embodiment of the present invention;

Fig. 6a is a cross-sectional view of a portion of a dosage form according to an alternate embodiment of the present invention;

Fig. 6b is a cross-sectional view of a portion of a dosage form according to an alternate embodiment of the present invention;

Fig. 6c is a cross-sectional view of a portion of a dosage form according to an alternate embodiment of the present invention;

Fig. 6d is a perspective view of a dosage form showing alphanumeric characters printed on the interior surface of the dosage form according to an alternate embodiment of the present invention;

Fig. 7 is a perspective view of a dosage form according to an alternate embodiment of the present invention;

Fig. 8 is a perspective view of a brachytherapy device according to an alternate embodiment of the present invention;

Fig. 9 is a flow chart of an exemplary method of making a dosage form according to an embodiment of the present invention.

### DESCRIPTION OF THE PREFERRED EMBODIMENTS

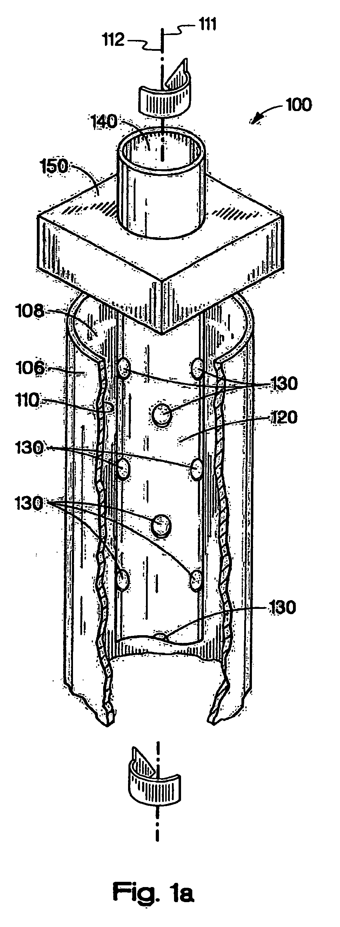

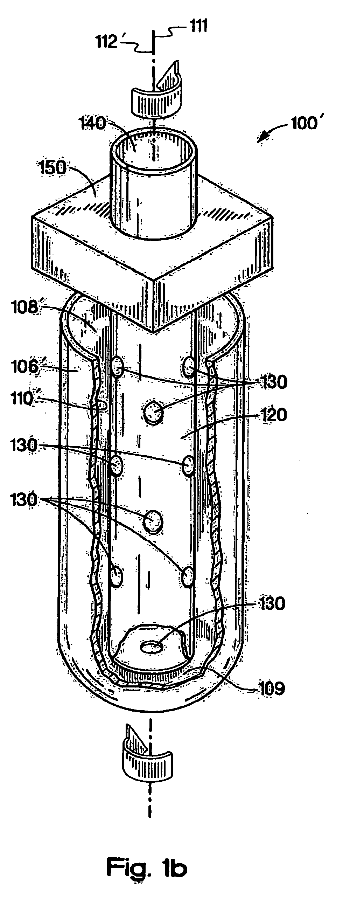

Referring to Fig. 1a, an embodiment of the present invention is shown in a perspective view. In this embodiment, fluid ejector head 100 includes fluid ejector body 120 adapted to be inserted into enclosing medium opening 108. Fluid ejector head 100 further includes nozzles 130 disposed on fluid ejector body 120 and fluidically coupled to fluid channel 140. Fluid ejector actuator 150 is in fluid communication with nozzles 130. Activation of fluid ejector actuator 150 ejects a bioactive fluid onto interior surface 110 of enclosing medium 106.

For purposes of this description and the present invention, the term enclosing medium may be any solid or semi-solid material with a shape, having a substantially fixed form, including an inside, or interior, surface and an outer, or exterior, surface. The term substantially fixed form is used to imply permanence of the interior surface of the object not of the shape of the object. For example, a bag may change shape depending on whether it is open or closed; however, the existence of the interior surface remains whether open or closed. In addition, the substantially fixed form also includes at least one opening having a cross-sectional area less than the maximum cross-sectional area obtainable for that shape. The enclosing medium may have rectangular parallelepiped, cylindrical, ellipsoidal, or spherical shapes just to name a few simple geometric shapes that may be utilized. For example, enclosing medium 106 may be a vial, a capsule, or a tube to name a few articles that may be utilized. In alternate embodiments, where enclosing medium 106 is, for example, a gelatin capsule or a vial having a bottom surface, fluid ejector head 100 may also include nozzles providing fluid ejection of the bioactive fluid onto bottom interior surface 109, as well as side interior surface 110' of enclosing medium 106 as shown in Fig. 1 b.

In this embodiment fluid ejector body 120 includes multiple bores or nozzles 130, the actual number shown in Figs. 1a and 1b is for illustrative purposes only. The number of nozzles utilized depends on various parameters such as the particular bioactive fluid to be deposited, the number of different bioactive fluids involved, the particular dosage to be generated, and the particular size of the enclosing medium to be utilized. In this embodiment, either fluid ejector body 120 or enclosing substrate 106 or both may be rotated about the longitudinal axis 112 of enclosing medium106. Fluid ejector head provides control of drug dosage by controlling drug particles on the inside or interior surface of an enclosing medium in a controlled manner.

For purposes of this description and the present invention, the term "bioactive" as used with fluid, composition, substance, or agent, is a composition that affects a biological function of a vertebrate directly or as a result of a metabolic or chemical modification associated with the vertebrate or its vicinal environment. An example of a bioactive fluid is a pharmaceutical substance, such as a drug, which is given to alter a physiological condition of the vertebrate, such as a disease. The term bioactive is meant to include any type of drug, medication, medicament, vitamin, nutritional supplement, or other compound that is designed to affect a biological function of a vertebrate. During the "printing" or deposition process, the bioactive substance or material will be present in a fluid, either because the bioactive is itself a fluid, or because the bioactive has been dissolved or suspended in another fluid.

It should be noted that the drawings are not true to scale. Further, various elements have not been drawn to scale. Certain dimensions have been exaggerated in relation to other dimensions in order to provide a clearer illustration and understanding of the present invention.

In addition, although some of the embodiments illustrated herein are shown in two dimensional views with various regions having depth and width, it should be clearly understood that these regions are illustrations of only a portion of a device that is actually a three dimensional structure. Accordingly, these regions will have three dimensions, including length, width, and depth, when fabricated on an actual device. Moreover, while the present invention is illustrated by various embodiments, it is not intended that these illustrations be a limitation on the scope or applicability of the present invention. It is not intended that the embodiments of the present invention be limited to the physical structures illustrated. These structures are included to demonstrate the utility and application of the present invention to presently preferred embodiments.

Fluid ejector body 120, in this embodiment, is a tubular shaped structure having an outside diameter less than the inside diameter of enclosing medium opening 108, such that fluid ejector body 120 is insertable into enclosing medium opening 108, along longitudinal axis 112, of enclosing medium 106. Enclosing medium 106 may be any medium having interior surface 110 utilized for drug delivery. In this embodiment, fluid ejector body 120 also includes a fluid ejector body longitudinal axis 111 that is aligned with longitudinal axis 112 of enclosing medium 106. In alternate embodiments, depending on various parameters such as the shape of the enclosing medium and the fluid ejector body, the fluid ejector body longitudinal axis may not be in alignment with the longitudinal axis of the enclosing medium. Fluid ejector body 120 may utilize any ceramic, metal, or plastic material capable of forming the appropriate sized tubular shape. Fluid ejector actuator 150 may be any device capable of imparting sufficient energy to the bioactive fluid either in fluid channel 140 or in close proximity to nozzles 130. For example, compressed air actuators, such as utilized in an airbrush, or electromechanical actuators or thermal mechanical actuators may be utilized to eject the bioactive fluid from nozzles 130.

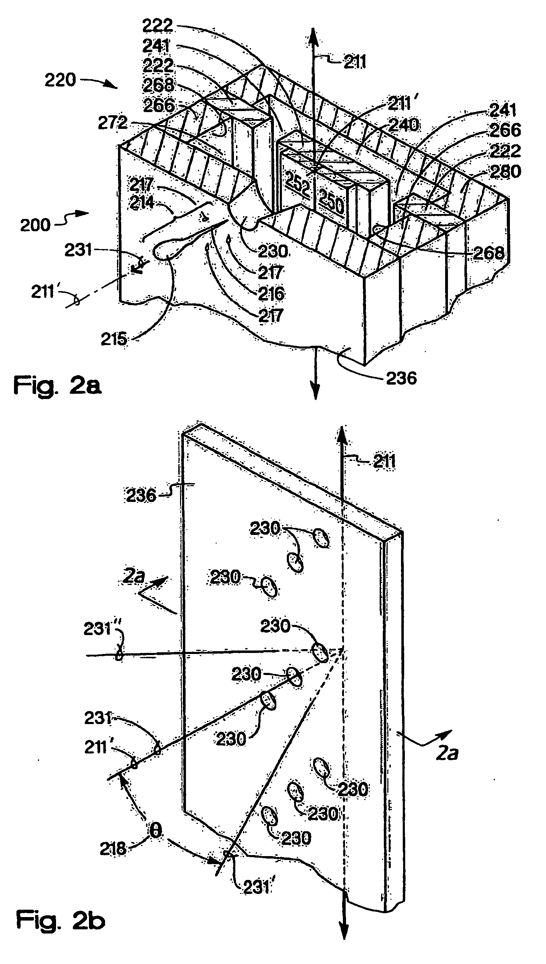

An exemplary embodiment of a fluid ejector head is shown in an isometric cross-sectional view in Fig. 2a. In this embodiment, fluid ejector head 200 includes fluid ejector body 220 wherein at least a portion of the body has a rectangular cross-section. In alternate embodiments, fluid ejector body may have a parallelepiped structure. In addition, nozzle 230 has an ejection axis 231 defining the general direction in which drops are ejected from fluid ejector body 220. Fluid body longitudinal axis 211 and nozzle ejection axis 231 form predetermined ejection angle 218 (see Fig. 2b). In this embodiment, nozzle ejection axis 231 may be aligned at an angle between 0° and 60° degrees from fluid body normal 211' of fluid body longitudinal axis 211 as shown in Fig. 2b. In alternate embodiments, nozzle ejection axis 232 is aligned at an angle between 0° and 45°, and more preferably nozzle ejection axis 232 is substantially perpendicular to fluid body longitudinal axis 211. In addition, ejection angles 231' and 231" illustrate that the angle may be either in a positive or in a negative direction relative to fluid body normal 211'.

Fluid ejector head 200 further includes fluid ejector actuator 250, chamber layer 266, fluid body housing 280, and nozzle layer 236. In this embodiment, substrate 222 is a portion of a silicon wafer. In alternate embodiments, other materials may also be utilized for substrate 222, such as, various glasses, aluminum oxide, polyimide substrates, silicon carbide, and gallium arsenide. Accordingly, the present invention is not intended to be limited to those devices fabricated in silicon semiconductor materials. In this embodiment, fluid body housing 280 and substrate 222 form fluid channel 240. Fluid inlet channels 241 are formed in substrate 222, and provide fluidic coupling between fluid channel 240 and chamber 272.

Fluid energy generating element 252 is disposed on substrate 222 and provides the energy impulse utilized to eject a bioactive fluid, sealant material, ink, or other suitable fluid from nozzle 230. As described above, fluid ejector actuator 250 may be any element capable of imparting sufficient energy to the bioactive fluid to eject it from nozzle 230. In this embodiment, fluid ejector actuator 250 includes fluid energy generating element 252, which is a thermal resistor. In alternate embodiments, other fluid energy generating elements such as piezoelectric, flex-tensional, acoustic, and electrostatic generators may also be utilized. For example, a piezoelectric element utilizes a voltage pulse to generate a compressive force on the fluid resulting in ejection of a drop of the fluid. In still other embodiments, fluid energy generating element 252 may be located some distance away, in a lateral direction, from nozzle 230. The particular distance will depend on various parameters such as the particular fluid being dispensed, the particular structure of chamber 272, and the structure and size of fluid channel 240, to name a few parameters.

The thermal resistor is typically formed as a tantalum aluminum alloy utilizing conventional semiconductor processing equipment. In alternate embodiments, other resistor alloys may be utilized such as tungsten silicon nitride, or polysilicon. The thermal resistor typically is connected to electrical inputs by way of metalization (not shown) on the surface of substrate 222. Additionally, various layers of protection from chemical and mechanical attack may be placed over the thermal resistor, but are not shown in Fig. 2a for clarity. Substrate 222 also includes, in this embodiment, one or more transistors (not shown for clarity) electrically coupled to fluid energy generating element 252. In alternate embodiments, other active devices such as diodes or memory logic cells may also be utilized, either separately or in combination with the one or more transistors. In still other embodiments, what is commonly referred to as a "direct drive" fluid ejector head, where substrate 222 may include fluid ejector generators without active devices, may also be utilized. The particular combination of active devices and fluid energy generating elements will depend on various parameters such as the particular application in which fluid ejector head 200 is used, as well as the particular fluid being ejected to name a couple of parameters.

In this embodiment, an energy impulse applied across the thermal resistor rapidly heats a component in the fluid above its boiling point causing vaporization of the fluid component resulting in an expanding bubble that ejects fluid drop 214 as shown in Fig. 2a. Fluid drop 214 typically includes droplet head 215, drop-tail 216 and satellite-drops 217, which may be characterized as essentially a fluid drop. In this embodiment, each activation of energy generating element 252 results in the ejection of a precise quantity of fluid in the form of essentially a fluid drop; thus, the number of times the fluid energy generating element is activated controls the number of drops 214 ejected from nozzle 230. Thus, fluid ejector head 200 may generate deposits of discrete droplets of a fluid, including, for example, a bioactive substance on the interior surface of an enclosing substrate or medium at controlled or predetermined locations on the interior surface of the enclosing medium. For example, when the fluid contains a bioactive solid substance dissolved or dispersed in a solvent, agglomeration of the bioactive particles, after evaporation of the solvent carrier, is hindered. By activating fluid energy generating element a predetermined number of times n, n bioactive fluid drops are ejected from nozzle 230. This embodiment provides for optimization of the bioactive substance for dissolution rate and dosage. It is further recognized that the fluid may include any solid material dissolved or suspended in one or more solvents.

The drop volume of fluid drop 214 may be optimized by various parameters such as nozzle bore diameter, nozzle layer thickness, chamber dimensions, chamber layer thickness, energy generating element dimensions, and the fluid surface tension to name a few. Thus, the drop volume can be optimized for the particular fluid being ejected as well as the particular application in which the enclosing medium will be utilized. Fluid ejector head 200 described in this embodiment can reproducibly and reliably eject drops in the range of from about 5 femtoliters to about 10 nanoliters depending on the parameters and structures of the fluid ejector head as described above. In alternate embodiments, fluid ejector head 200 can eject drops in the range form about 5 femtoliters to about 1 microliter. In addition, according to other embodiments, multiple fluid ejector heads 200 may be ganged together to form polygonal structures. For example, two fluid ejector heads 200 may be formed back to back providing the ability to dispense multiple bioactive fluids or any combination of bioactive fluids, ingestible inks, and sealants. Further, fluid ejector head 200 may also contain a fluid that is a mixture of bioactive fluid, ingestible ink, and sealant or various combinations thereof. The term fluid includes any fluid material such as bioactive substances, inks, chemical or biological reagents, as well as fluids containing dissolved or dispersed solids in one or more solvents.

Chamber layer 266 is selectively disposed over the surface of substrate 222. Sidewalls 268 define or form fluid ejection chamber 272, around energy generating element 252, so that fluid, from fluid channel 240 via fluid inlet channels 241, may accumulate in fluid ejection chamber 272 prior to activation of energy generating element 252 and expulsion of fluid, through nozzle or orifice 230 when energy generating element 252 is activated. The fluid chambers formed along longitudinal axis 211 may be in a straight line or a staggered configuration depending on the particular application, in which fluid ejector head 200 is utilized, a staggered configuration is illustrated in Fig. 2b. Nozzle or orifice layer 236 is disposed over chamber layer 266 and includes one or more bores or nozzles 230 through which fluid is ejected. In alternate embodiments, depending on the particular materials utilized for chamber layer 266 and nozzle layer 236 an adhesive layer may also be utilized to adhere nozzle layer 236 to chamber layer 266. According to additional embodiments, chamber layer 266 and nozzle layer 236 are formed as a single integrated chamber nozzle layer. Chamber layer 266, typically, is a photoimagible film that utilizes photolithography equipment to form chamber layer 266 on substrate 222 and then define and develop fluid ejection chamber 272.

Nozzle layer 236 may be formed of metal, polymer, glass, or other suitable material such as ceramic. In this embodiment, nozzle layer 236 is a polyimide film. Examples of commercially available nozzle layer materials include a polyimide film available from E. I. DuPont de Nemours & Co. sold under the name "Kapton", a polyimide material available from Ube Industries, LTD (of Japan) sold under the name "Upilex." In an alternate embodiment, the nozzle layer 236 is formed from a metal such as a nickel base enclosed by a thin gold, palladium, tantalum, or rhodium layer. In other alternative embodiments, nozzle layer 236 may be formed from polymers such as polyester, polyethylene naphthalate (PEN), epoxy, or polycarbonate.

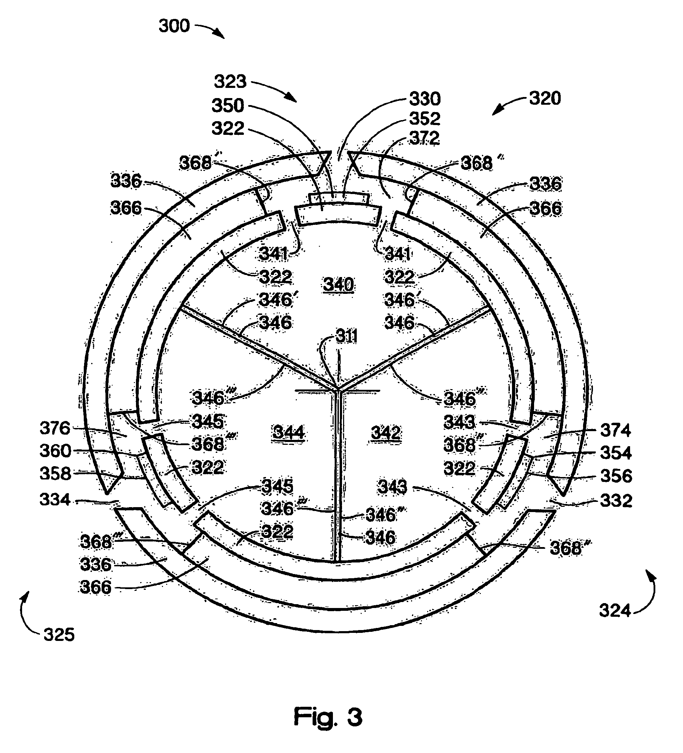

An alternate embodiment of a fluid ejector head is shown in a cross-sectional view in Fig. 3. In this embodiment, fluid ejector head 300 includes fluid ejector body 320 wherein at least a portion of the body has a cylindrical cross-sectional shape, including fluid body longitudinal axis 311 projecting in and out of the cross sectional view. In alternate embodiments fluid ejector body 320 may have a portion having a curvilinear shape. Fluid ejector head 300 further includes fluid ejector actuator 350, ink ejector actuator 354, and sealant ejector actuator 358 disposed on fluid ejector body 320. Although the fluid ejector actuators are disposed under the nozzles in this embodiment, in alternate embodiments, the fluid ejector actuators may be positioned some lateral distance away from the nozzles. The particular distance will depend on various parameters such as, the particular fluid being dispensed, the particular structure of the chambers, and the structure and size of the fluid channels, to name a few parameters. Fluid channel separator 346 is attached to substrate 322 and separates fluid ejector head 300 into three sections: fluid section 323, ink section 324, and sealant section 325. In this embodiment, fluid channel 340 is formed by fluid channel separator portions 346' and substrate 322; ink channel 342 is formed by fluid channel separator portions 346" and substrate 322; and sealant channel 344 is formed by fluid channel separator portions 346''' and substrate 322.

Fluid inlet channels 341 provide fluidic coupling between fluid channel 340 and chamber 372, and are formed in substrate 322 within fluid section 323. Fluid inlet channels 343 and 345 provide fluidic coupling between fluid channels 342 and 344 and chambers 374 and 376 respectively. Fluid energy generating element 352 is disposed on substrate 322 and provides the energy impulse utilized to eject fluid from nozzle 330. Fluid energy generating elements 356 and 360 provide the energy impulses utilized to eject fluid from nozzles 332 and 334 respectively. In this embodiment, fluid energy generating elements 352, 356, and 360 are thermal resistors that rapidly heat a component in the fluid above its boiling point causing vaporization of the fluid component resulting in ejection of a drop of the fluid. In alternate embodiments, other fluid energy generating elements such as piezoelectric, flex-tensional, acoustic, and electrostatic generators may also be utilized. In this embodiment, fluid energy generating elements 352, 356, and 360 eject the fluid in a substantially radial direction onto the interior surface of the enclosing medium (not shown).

Chamber layer 366 is disposed over substrate 322 wherein sidewalls 368' define or form a portion of fluid ejection chamber 372 in fluid section 323; sidewalls 368" form a portion of ink ejection chamber 374 in ink section 324; and sidewalls 368''' form a portion of sealant ejection chamber 376 in sealant section 325. Nozzle or orifice layer 336 is disposed over chamber layer 366 and includes one or more bores or nozzles 330, 332, and 334 through which fluid, in the three sections, is ejected. In alternate embodiments, depending on the particular materials utilized for chamber layer 366 and nozzle layer 336, an adhesive layer may also be utilized to adhere nozzle layer 336 to chamber layer 366. According to additional embodiments, chamber layer 366 and nozzle layer 336 are formed as a single layer. Such an integrated chamber and nozzle layer structure is commonly referred to as a chamber orifice or chamber nozzle layer.

Although Fig. 3 depicts fluid ejector body 320 separated into three sections, alternate embodiments may utilize anywhere from a single section to multiple sections depending on the particular application in which fluid ejector head 300 is utilized. For example, fluid ejector body 320 may have a single section to eject a bioactive fluid. In addition, the fluid chambers formed along longitudinal axis 311 may be in a straight line, staggered configuration, or helical configuration depending on the particular application in which fluid ejector head 300 is utilized. In another example, fluid ejector body 320 includes six sections having straight, staggered, or helical configurations, providing for any of the possible combinations of dispensing multiple bioactive fluids, inks, and sealants depending on the particular application in which fluid ejector head 300 is utilized. In an alternate embodiment, each section may eject a fluid having a bioactive component, ink component and sealant component or any combination thereof.

In addition to having various numbers of sections each section may also be independently optimized for performance. For example, the energy generating elements of each section may be optimized for the particular fluid ejected by that section. Further, the dimensions of the ejection chambers and nozzles may also be optimized for the particular fluid ejected by that section. Energy generating elements as well as chamber and nozzle dimensions within a section may also be varied providing ejection of different drop sizes of the same fluid to be ejected from fluid ejector head 300.

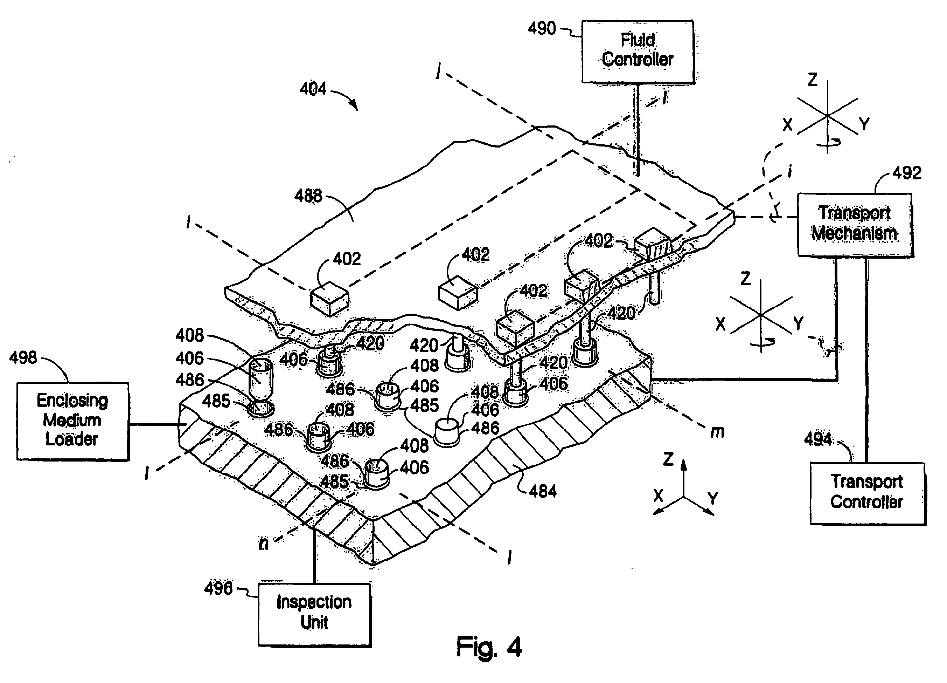

Referring to Fig. 4 an exemplary embodiment of bioactive fluid dispensing system 404 of the present invention is shown in a perspective view. In this embodiment bioactive fluid dispensing system 404 includes enclosing medium tray 484 having an n x m array of enclosing medium holders 486 adapted to accept insertion of enclosing medium parts 406. Bioactive fluid dispensing system 404 further includes an i x j array of fluid ejection cartridges 402 that include ejector bodies 420 adapted to be inserted into enclosing medium openings 408. For example, a system may utilize a tray having a 4 x 4 array of holders containing enclosing medium parts and a 2 x 2 array of fluid ejector bodies wherein the tray is effectively divided into four sections of 2x2 holders and the fluid ejector bodies are inserted in the enclosing medium parts in each section. In this embodiment, the array of fluid ejection cartridges 402 is mounted to dispensing bracket 488. Fluid ejector actuators (not shown) are operably coupled to fluid ejector bodies 420 and fluid controller 490, such that fluid controller 490 activates fluid the ejector actuators, to eject, for example, a bioactive fluid at controlled or predetermined locations onto the interior surface of enclosing medium parts 406. In addition, fluid controller 490 is operably coupled to a rotation mechanism (not shown) disposed on fluid ejection cartridges 402 to rotate fluid ejector bodies 420 about a fluid body longitudinal axis (not shown).

Transport mechanism 492 is coupled to either dispensing bracket 488 or enclosing medium tray 484 or both depending on the particular application in which dispensing system 404 is utilized. Transport mechanism 492 is operably coupled to transport controller 494, and provides signals controlling movement of enclosing medium tray 484 to align enclosing medium openings 408 to fluid ejector bodies 420 as well as insert and withdraw fluid ejector bodies 420 from enclosing medium parts 406. For example, transport mechanism 492 may move enclosing medium tray 484 in X and Y lateral directions while raising and lowering (i.e. movement in the Z direction) dispensing bracket 488 to withdraw and insert fluid ejector bodies 420 into enclosing medium parts 406 as shown in Fig. 4. In alternate embodiments, other combinations of movements may be utilized and controlled by transport mechanism 492. such as rotation of enclosing medium tray 484 about a central axis to provide additional alignment motion. In this embodiment, fluid controller 490 and transport controller 494 may utilize any combination of application specific integrated circuits (ASICs), microprocessors and programmable logic controllers to control the carious functions of fluid dispensing system 404. The particular devices utilized will depend on the particular application in which fluid dispensing system 404 is utilized. In addition, dispensing system 404 may optionally include an enclosing medium loader 498 to load enclosing medium parts 406 into enclosing medium holders 486. Further, dispensing system 404 may also include enclosing medium rotator 485 to rotate enclosing medium parts 406 around an enclosing medium longitudinal axis (see Fig. 1a and 1b) thus rotate the interior surface of the enclosing medium around the fluid ejector body. Either rotation of enclosing medium parts 406 or rotation of fluid ejector bodies 420 or both can be utilized to generate a two-dimensional array of discrete deposits dispensed onto the interior surface of enclosing medium parts 406.

Optional inspection unit 496 may be utilized to provide on-line, non-destructive quality assurance testing of the manufactured dosage forms. Monitoring the amount of active substance deposited onto enclosing medium parts 406 may be carried out. For example, near infrared or other optical technique may be utilized to perform a rapid in line assay of the active agent or agents onto enclosing medium parts 406. Such in line testing can be performed before various excipients, if desired, are added, and thus removes overlap or interference of the excipient signal in the assay measurement. In addition inspection unit 496 may also be utilized to optically monitor the quality of characters generated on the interior surface of enclosing medium parts 406.

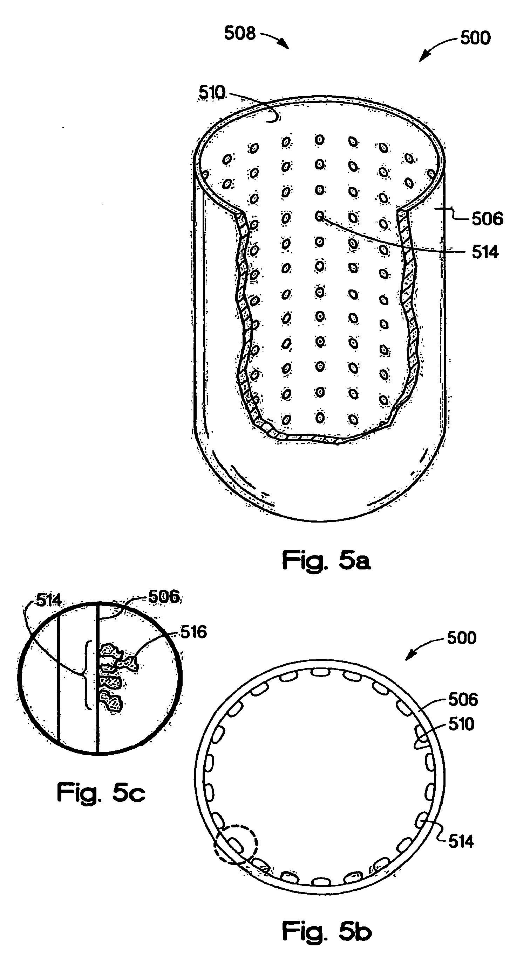

Referring to Fig. 5a, an exemplary embodiment of enclosing medium 506 of the present invention is shown in a perspective view as a pharmaceutical dosage form 500. In this embodiment, dosage form 500 is a gelatin capsule on which discrete deposits 514 are deposited on inside surface 510 of dosage form 500. Discrete deposits 514 may be in lines with uniform spacing as shown, or may be in any desired two or three dimensional arrangement. Dosage form 500 can be any ingestible material that dissolves or degrades in body fluids, or enzymes or both. Dosage from 500 also may be insoluble or relatively insoluble but allow entry of liquids by either diffusion or via perforations. The liquid may then promote drug release from dosage form 500 either by diffusion or escape through the perforations. Typically dosage form 500 is hydrophilic and readily disintegrates in water, and in other embodiments, the dissolution or disintegration of dosage form 500 is enhanced at the pH of the fluids in the stomach or upper intestine. Dosage form 500 may be coated to promote or resist dissolution at specific sites in the gastrointestinal tract. In addition, ingestible materials that minimize unintended interactions with the bioactive fluid dispensed in dosage form 500 are desirable. Further, ingestible materials that minimize the release of a component, that would cause unintended interactions with the bioactive active agent, included in the dispensed fluid, upon dissolution of dosage form 500 are also desirable. The ability to remain stable over extended periods of time, at elevated temperatures, and at high or low levels of relative humidity are additional properties of dosage form 500 that are desirable. In addition, in still other embodiments, gelatin capsule 508 is generally a poor medium for the growth of microorganisms to reduce spoilage. Three examples of exemplary dosage forms are capsules made of gelatin, capsules made of (hydroxypropyl) methyl cellulose (HPMC), or capsules made out of enteric coating materials. The dosage forms may be coated over the outside surface with one or more materials as is useful, or desirable, to improve appearance, or function, such as ease of swallowing, or to modify, or influence release of the bioactive agent. Typically, gelatin capsule 508 has an outer diameter in the range from about 3 mm to about 12 mm, however, depending on the particular bioactive fluid being dispensed other sizes may also be utilized. For example, larger sizes are common in veterinary medicine.

In this embodiment, discrete deposits 514 are deposited, in predetermined locations forming an array of bioactive agent particles, on inside surface 510 of enclosing medium 506. The deposition of solutions of a bioactive agent, dissolved, dispersed, or suspended in a solvent or mixture of solvents, in discrete deposits 514 generates microparticulate dispersions after the solvent has been removed or absorbed by the capsule. A cross-sectional view of gelatin capsule 508 is shown in Fig. 5b illustrating a row of discrete deposits 514 deposited around the inside circumference of capsule 508. In an alternate embodiment, discrete deposits 514 may form several arrays of different bioactive agents. For example, the capsule may contain four different bioactive agents dispersed so that every fourth discrete deposit contains the same agent or every fourth row or column would contain the same agent, or any combination with different amounts of different bioactive agents. Another example would be where capsule 508 contains four sections, each section containing discrete deposits 514 of a particular bioactive agent. An expanded cross-sectional view of discrete deposits 514 is shown in Fig. 5c showing microparticulates 516 formed on inside surface 510 of capsule 508 after the solvent has been removed or absorbed by the capsule. In an alternate embodiment, capsule 508 may be, for example, an enteric coated capsule for those applications in which the bioactive agent or agents deposited are to be released in the intestine.

The bioavailability, or in other terms the rate and extent of absorption, of a bioactive agent is often related to the rate of dissolution of the bioactive agent. The rate of dissolution is dependent on particle size and typically given similar conditions the rate of dissolution of a particular agent increases as the particle size decreases. Thus by utilizing a fluid ejector head adapted to be inserted into the opening of a capsule solutions, dispersions, or suspensions of a bioactive agent may be deposited as fluid drops to form microparticulates on the surface of the capsule. In this embodiment the size of these fluid drops can be controlled, as described above, and are typically in the range from about 5 femtoliter to about 10 nanoliters, which corresponds to microparticulate deposits in the picogram to microgram range depending on the ratio of the amount of bioactive agent to the amount of solvent in a drop. In alternate embodiments, the size of these fluid drops can be in the range from about 5 femtoliter to about 1 microliter, which corresponds to microparticulate deposits in the picogram to milligram range depending on the ratio of the amount of bioactive agent to the amount of solvent in a drop. Deposition of discrete deposits 514 also hinders agglomeration of microparticulates 516.

The bioactive fluid may be formulated so discrete deposits 514 on inside surface 510 of capsule 508 may be known types of pharmaceutical formulations such as eutectic mixtures, liposomes, emulsions, amorphous co-precipitates, micronized drug particles, solid-in-solid solution, glassy state powders, co-solvent solutions, and solutions in polymers as only some examples. Further, for bioactive agents that are released in the stomach the dosed capsule 508 does not require being filled with excipients and will then float on gastric contents while the capsule and bioactive agent dissolve, increasing the amount of bioactive agent released in the stomach. For example, a hydrophobic excipient, may include a waxy sealant with a density less than 1.0 gram per milliliter, which may be applied over discrete deposits 514, this "inside liner material" will help the capsule float in the stomach, and release drug in the stomach, while the capsule shell and bioactive material are dissolving. In still other embodiments an excipient or "inside liner material" may be other polymeric materials such as a diffusional resistant membrane or an enteric coating membrane. In addition, the utilization of such an excipient "inside liner material" may also be used to generate three-dimensional arrays of, a bioactive substance or multiple bioactive substances on inside surface 510 of enclosing medium 506. For example, the enclosing medium may be a gelatin capsule with a bioactive agent deposited on the interior surface of the capsule and then an enteric coating as the "inside liner material" deposited over the bioactive agent deposits. By repeating this process for the desired number of doses a pulsed release of the bioactive agent may be obtained, as for example in hyper-kinetic children for Ritalin^{®} as just one example.

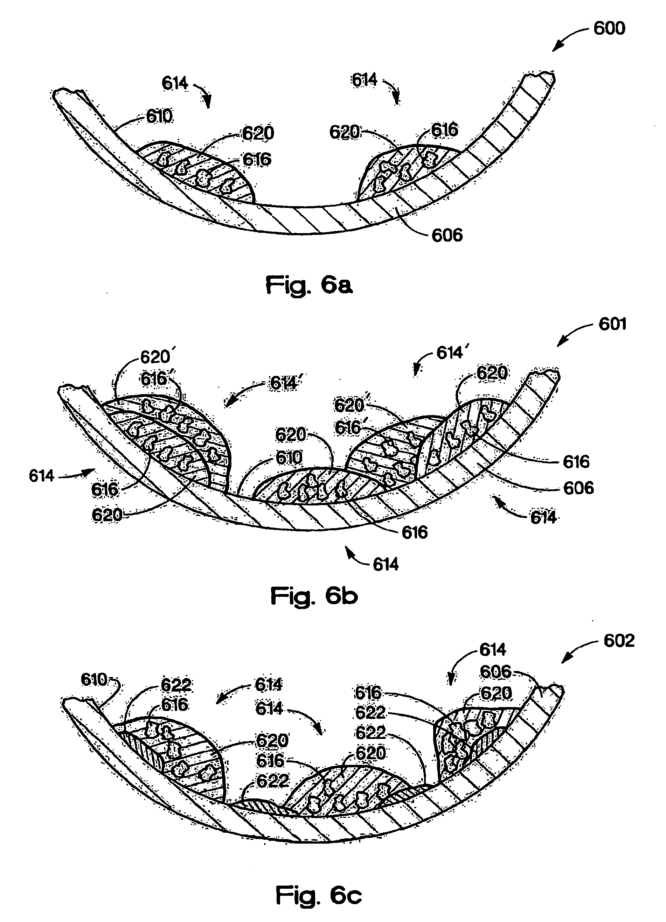

An alternate embodiment of the present invention in an expanded cross-sectional view of a portion of dosage form 600 is shown in Fig. 6a. In this embodiment, sealant material 620 is deposited over discrete deposits 614 which were deposited on inside surface 610 of enclosing medium 606 forming dosage form 600. Discrete deposits 614 generate microparticulates 616, similar to that described for Fig. 5. Sealant or barrier material 620 acts to seal the bioactive agent from the environment. Depending on the particular bioactive agent dispensed, and the particular dosage form material used, the barrier material provides various protective properties, such as humidity protection, protection from oxidation, inactivation, or contamination. The sealant or barrier material also further limits agglomeration. The sealant or barrier material is an edible coating made from a suitable polymeric material such as, for example, a water-soluble polyoxyethylene or cellulose ether derivative, or waxy material.

An alternate embodiment of the present invention in an expanded cross-sectional view of a portion of dosage form 601 is shown in Fig. 6b. In this embodiment, the deposition of multiple deposits of the same or different bioactive agents on dosage form 601 is illustrated. Discrete deposits 614 generate microparticulates 616 and sealant material 620 is similar to that described above. Discrete deposits 614' forming microparticulates 616' and sealant material 620' are deposited after sealant material 620 is deposited. Thus, microparticulates 616' and sealant material 620' create a dosage form having either an increased dosage of a particular bioactive agent or a dosage form having multiple bioactive agents deposited therein. Such a process can be repeated to provide increasing dosages or complex dosage forms with many bioactive agents, with the same or different release and absorption patterns. In addition sealant materials 620 and 620' can be the same or different depending on the particular application in which dosage form 601 will be utilized as well as the properties and compatibilities of the bioactive agent or agents being used. For example, sealant materials 620 and 620' may be formulated to provide controlled release of a particular bioactive agent. Another example, would be where sealant materials 620 and 620' may be formulated to provide separation of a particular active ingredient from other bioactive agents that are physically or chemically incompatible. Controlled release in this context is where the bioactive agent dissolution rate or release rate may be fast, slow, sustained, or pulsed. Thus, pharmaceutical dosage forms may be generated that release an increasing, decreasing, constant, pulsed at uniform times, or pulsed at varying times, amount of a bioactive substance over time, after being ingested.

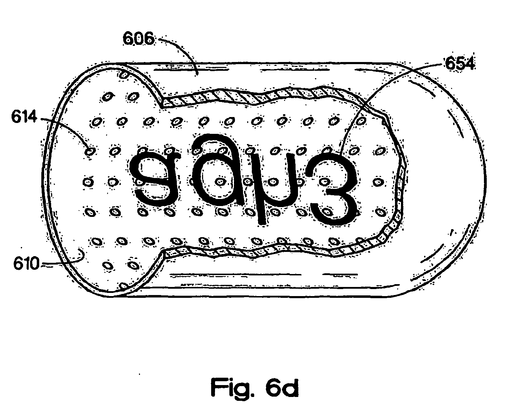

An alternate embodiment of the present invention in an expanded cross-sectional view of a portion of dosage form 602 is shown in Fig. 6c. In this embodiment, ink deposits or dots 622 are deposited on inside surface 610 of enclosing medium 606 in patterns using dot matrix manipulation or other means to generate an image, alphanumeric characters, or a machine understood code such as a one or two dimensional bar code, on enclosing medium 606. Discrete deposits 614 and sealant material 620 is deposited over ink deposits 622. For example, ink deposits 622 generate the alphanumeric characters "agh3" printed on the inside of enclosing medium 606 in reverse letters to be legible from the outside, as shown in Fig. 6d.
Such characters can be utilized to meet Federal Drug Administration requirements for drug capsule identification by printing on the inside of enclosing medium 606, such characters or images are not as easily rubbed off or washed off as for conventional dosage forms printed on the outside surface. In alternate embodiments ink deposits 622 may be deposited after or between discrete deposits 614 and sealant material 620. In other embodiments, multiple bioactive agents may also be deposited before or after ink deposits 622. Enclosing medium 606, in this embodiment, is either clear or sufficiently transparent to detect or observe ink dots 622. In addition, a bioactive agent can also be included in the ink formulation if desired.

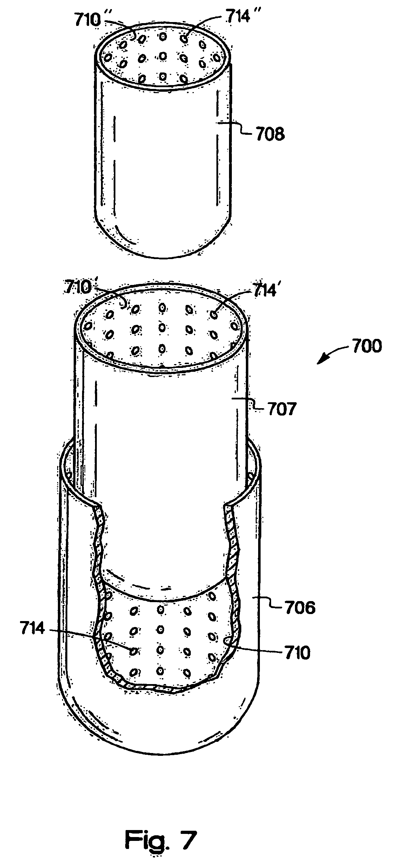

An alternate embodiment of a pharmaceutical dosage form of the present invention is shown in a perspective view in Fig. 7. In this embodiment, discrete deposits 714 are deposited, in predetermined locations forming an array of bioactive agent particles, on interior surface 710 of capsule 706 as described above and shown in Figs. 5 and 6. Dosage insert 707 has discrete deposits 714' deposited on interior surface 710' forming a second array of bioactive agent particles. Dosage insert 707 has an outer diameter less than the inside diameter of capsule 706 so that dosage insert 707 is insertable into capsule 706. Dosage insert 708 has discrete deposits 714" deposited on interior surface 710" forming a third array of bioactive agent particles. Dosage insert 708 has an outer diameter less than the inside diameter of dosage insert 707 so that dosage insert 708 is insertable into dosage insert 707. As described above dosage inserts 707 and 708, in alternate embodiments, may have multiple bioactive agents deposited in various combinations as previously described. In this embodiment, the combination of capsule 706 and dosage inserts 707 and 708 form dosage form 700 having a bioactive substance gradient in the radial direction of the dosage inserts and capsule 706. In this manner by varying the areal denisty of bioactive substance deposits in each insert, dosage forms may be generated, that after being ingested the gradient in the amount of bioactive substance released over time may increase, decrease, remain constant, pulsed at different times, or pulsed at constant times. In an alternate embodiment, dosage inserts 707 and 708 may include a bioactive agent or agents deposited on the outer surface as well. Further, multiple inserts greater than three can also be utilized. And in still other embodiments, capsule 706, and dosage inserts 707 or 708, or any combination thereof may be enteric capsules or gelatin capsules.

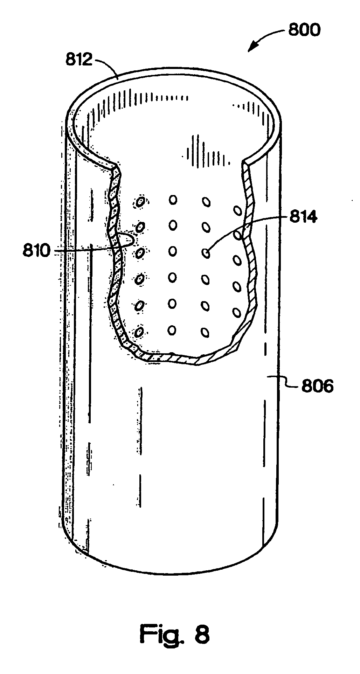

Referring to Fig. 8 an exemplary embodiment of enclosing medium 806 of the present invention is shown in a perspective view as a radiation-emitting element 800. In this embodiment, radiation-emitting element 800 is a tube on which discrete deposits 814 of a radioactive material are deposited on interior surface 810. Tube 812, in this embodiment, is typically less than about 1 mm in diameter, and is typically a metal such as titanium. A predetermined amount and pattern, as previously discussed in earlier embodiments, of a radioactive fluid may be deposited on the interior surface of enclosing medium 806. Such a device may be utilized in the localized treatment of tumors and other medical conditions by the interstitial implantation of radiation-emitting element 800. Such radioactive implants may be utilized to provide radiation therapy to destroy, reduce, or prevent the growth of tumors. The direct implantation of such radioactive sources directly into solid tumors is generally referred to as brachytherapy. The radioisotope to be deposited may be either dissolved as a salt or suspended, in an appropriate solvent or solvents. Examples of radioisotopes that may be utilized are palladium-103, iodine-125, gold-199, ytrium-90 iridium-192 and americium-241 to name a few. After the particular dose and pattern have been generated, the solvent is removed typically by heating and then the ends of tube 812 are sealed. For example, the ends may be crimped or welded closed. In alternate embodiments, a sealant material by be applied with or over the radioisotope.

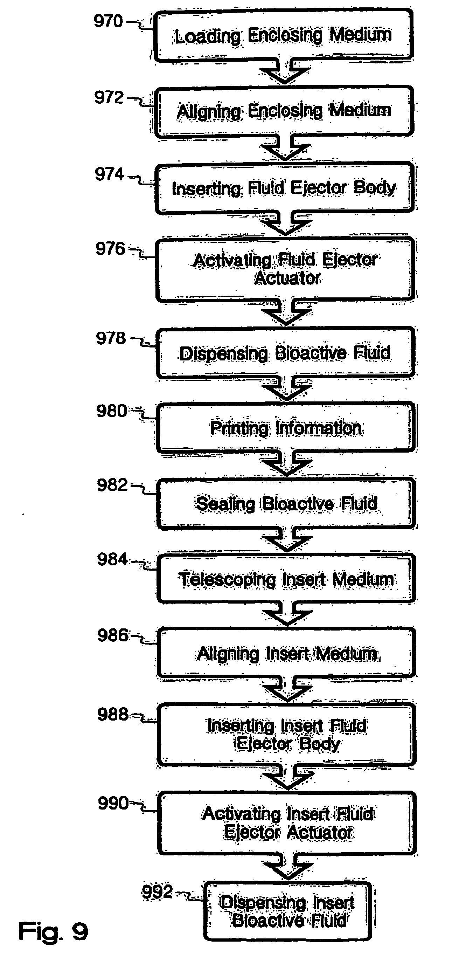

An exemplary embodiment of a method for generating a dosage form where the bioactive substance is deposited onto the inside surface of an enclosing medium is shown as a flow diagram in Fig. 9. Loading enclosing medium process 970 is utilized to position the enclosing medium to accept insertion of the fluid ejector body into an opening of the enclosing medium. Typically, this process may utilize any of the conventional loading techniques such as pick-and-place technologies or utilizing a hopper and shaker table, to load the enclosing medium onto a pallet or tray, as just a couple of examples. In one embodiment, the pallet or tray is loaded in-line on the bioactive dispensing system. However, in alternate embodiments, depending on the particular bioactive fluid dispensing system utilized, loading enclosing medium process 970 may be performed external to the bioactive fluid dispensing system, and the filled tray or pallet loaded onto the fluid dispensing system.

Aligning enclosing medium process 972 is used to align the opening in the enclosing medium to the fluid ejector head so that the fluid ejector body may be inserted into the enclosing medium. The tray holding the enclosing medium is typically under the control of a tray position controller or transport controller. Any of the conventional techniques for aligning parts may be utilized. For example, an electric or pneumatic motor or other actuator may move the tray in an X and Y direction to establish proper alignment of the enclosing medium to the fluid ejector head. In addition, typically a theta or rotational alignment about a Z-axis will also be provided. Further, sensors located on the tray, or an optical vision system or combination thereof will, typically, be utilized to provide feed back that the tray and the enclosing medium is properly aligned to the fluid ejector body. In alternate embodiments, tray position controller may be linked to a cartridge or fluid ejector head controller, or to a dispensing bracket holding the fluid ejection cartridges or fluid ejector heads, providing movement of the fluid ejector body or both the fluid ejector body and the tray to properly align the enclosing medium to the fluid ejector heads.

Inserting fluid ejector head process 974 is utilized to insert the fluid ejector body into the opening of the enclosing medium. The fluid ejector head is typically under the control of a cartridge or a fluid ejector head position controller or transport mechanism and transport controller. For example, in one embodiment, an electric or pneumatic motor may raise and lower, in the Z direction, the fluid ejector head providing the movement for inserting the fluid ejector body into the opening of the enclosing medium. In alternate embodiments the tray or a combination of the tray and the fluid ejector head are moved to insert the fluid ejector head into the opening of the enclosing medium.

Activating fluid ejector actuator process 976 is utilized to eject, for example, a bioactive fluid from at least one nozzle disposed on the fluid ejector head. Typically, a drop-firing controller or fluid controller in the bioactive fluid dispensing system, coupled to the fluid ejector head, activates the fluid ejector actuator, of the fluid ejection cartridge or fluid ejector head, to eject fluid drops containing the bioactive substance. For those embodiments, utilizing a fluid energy generating element, such as piezoelectric or thermal resistor elements, the drop firing controller will typically activate a plurality of fluid energy generating elements to eject essentially a drop of the bioactive fluid for each fluid energy generating element activated. Thus, by activating fluid energy generating element a predetermined number of times n, n bioactive fluid drops are ejected. In this embodiment, the fluid energy generating elements can reproducibly and reliably eject drops in the range of from about 5 femtoliters to about 10 nanoliters. However, in alternate embodiments, the size of these drops can be controlled, in the range from about 5 femtoliters to about 1 microliter. Such a drop size corresponds to microparticulate deposits in the picogram to milligram range depending on the ratio of the amount of bioactive substance to the amount of solvent in the fluid drop ejected.

Dispensing bioactive fluid process 978 is utilized to dispense and control the location of the ejected bioactive fluid drops on the inside surface of the enclosing medium to form the discrete bioactive substance deposits. Depending on the particular fluid ejector head utilized the bioactive fluid drops may be ejected through the nozzles along a nozzle ejection axis at a predetermined ejection angle from a fluid body normal. During dispensing bioactive fluid process 978 the fluid body normal and the surface normal of the enclosing medium are substantially parallel, resulting in the bioactive fluid drops being ejected at a predetermined angle to the surface normal of the enclosing medium. In one embodiment the nozzle ejection axis is aligned at an angle between about 0° and about 60° from the fluid body normal. In alternate embodiments, a fluid ejector head having a nozzle ejection axis aligned at an angle between about 0° and about 45° from the fluid body normal may be utilized. Preferably, a fluid ejector head with a nozzle ejection axis substantially perpendicular to a fluid ejector body longitudinal axis is utilized.

In addition, depending on the particular fluid ejector body utilized dispensing bioactive fluid process 978 may also include an optional rotational displacement process. The rotational displacement process is utilized to create rows of the discrete bioactive agent deposits for those embodiments utilizing fluid ejector heads having a single column of nozzles for a particular bioactive fluid. By utilizing rotation, dispensing bioactive fluid process 978 may generate a two-dimensional array forming an areal density of bioactive agent deposits on the interior surface of the enclosing medium. In addition, alternating between deposition of discrete deposits of bioactive fluid and deposition of discrete deposits of a barrier material over the dispensed bioactive fluid a three-dimensional array may be generated. Further by modifying the areal density in each layer a gradient in the dispensed bioactive substance, in the radial direction of the enclosing medium, can be formed. In addition, for those embodiments utilizing fluid ejector heads having multiple columns of nozzles the rotational displacement may be utilized to form rows of the discrete bioactive agent deposits having a smaller spacing between deposits than obtained with the same fluid ejector head without rotation. The rotational displacement may be accomplished by any of the conventional techniques utilized for rotation such as electrical or pneumatic motors, or piezoelectric motors to name just a couple of examples. The rotational displacement may be imparted to the enclosing medium, to the fluid ejector body, or some combination thereof.

Dispensing bioactive fluid process 978 may also include an optional vertical displacement process. The vertical displacement process may be utilized to create columns of the discrete bioactive agent deposits having a smaller spacing between deposits than normally obtained with the same fluid ejector head without vertical displacement. The fluid drop controller typically controls the vertical displacement, however a separate controller may also be utilized. For example, the fluid drop controller may be coupled to the tray position controller or the fluid ejector head controller or both to generate the appropriate vertical displacement. In alternate embodiments, separate controllers and motors or other actuators may be utilized to generate the appropriate vertical displacement. A dosage form is generated when the required number of fluid drops of the bioactive fluid, to create the desired pharmaceutical dose, have been dispensed on at least a portion of the enclosing medium. Depending on the particular fluid ejector body utilized, the dosage form may contain a two or a three-dimensional array of the discrete deposits of the bioactive substance on the inside surface of the enclosing medium. However, other arrangements can also be utilized, such as overlapping deposits forming essentially a layer, or a different geometrical arrangement of the deposits. For example a three dimensional array of discrete deposits may be generated by alternating between deposition of a bioactive fluid and a sealant material.

Optional printing information process 980 is utilized to print information on the enclosing medium. The information may be printed either on the inside or outside surface of the enclosing medium. By utilizing an insertable fluid ejector body as described above information may be printed on the inside surface of the enclosing medium. In one embodiment, a mixture of a bioactive fluid and an ingestible ink; may be utilized. However, in alternate embodiments, a separate set of ink ejector actuators or ink energy generating elements on the fluid ejector body may also be utilized to eject drops of an ingestible ink, to deposit ink dots in patterns on the inside surface of the enclosing medium. In still other embodiments, separate fluid ejector heads may also be utilized. For example, either before or after deposition of the bioactive fluid, the tray holding the enclosing medium may be transported to a printing station. At the printing station an ink ejector body may be inserted into the opening of the enclosing medium and an ink ejector actuator is activated to eject drops of an ingestible ink. Dot matrix manipulation or other means, coupled with rotation and optionally vertical displacement, may be utilized to generate an image, alphanumeric characters, or a machine understood code such as a one or two-dimensional bar code, on the enclosing medium.

In printing information process 980, information may be printed either in a machine understood form or it can be printed in a human perceptible form or in some combination thereof. Appropriate manufacturing information, such as required by the FDA may be printed on the enclosing medium. User or patient information, such as the name of the user or patient and the date and time for administering the dosage form, may also be printed on the enclosing medium. Depending on various parameters such as the particular bioactive agent or agents being deposited, the particular composition of the enclosing medium, as well as the composition of the ingestible ink, printing information process 980, may be carried out at various times in the process of generating a dosage form. For example, the ingestible ink may be deposited before the bioactive fluid and covered with a barrier material. Another example would include depositing the bioactive fluid, covering the bioactive fluid with a barrier material and then printing over the barrier material. Still another example would include the bioactive fluid deposited directly over the ingestible ink.

Optional sealing bioactive fluid process 982 is utilized to cover or seal the bioactive fluid deposits with a barrier material that protects or separates the bioactive agent or agents deposited from other agents or materials included in the dosage form. For example, dosage forms may contain multiple deposits of different bioactive agents dispensed over the same area wherein each agent is separated from the other agents by the barrier material. Another example is that described above utilizing sealing bioactive fluid process 982 to deposit a barrier material over an ingestible ink. By sealing a bioactive agent or other material with the barrier material, dosage forms may contain bioactive agents that are physically or chemically incompatible.

The barrier material provides various protective properties, such as humidity protection, protection from oxidation, inactivation, or contamination. The barrier material is an ingestible coating made from a suitable polymeric material such as a water-soluble polyoxyethylene or cellulose ether derivative, waxy material or other suitable substance. In addition, typically the barrier material is an inert material, which will not interact with the deposited bioactive fluid. However, in alternate embodiments, the barrier material may be designed (i.e. will contain additives) to specifically interact with the bioactive fluid dispensed. For example, the barrier material may contain an ion that will form a salt with the bioactive substance. In another example, an additive in the barrier material may interact or react with a solvent or multiple solvents to solidify the dispensed solvents but not interact or react with the active ingredient. In this embodiment, the fluid ejector actuators activated may be a different subgroup of fluid ejector actuators on the fluid ejector body used to dispense the bioactive fluid as shown in Figs. 3-4, or a different fluid ejector body may also be utilized. For example, a fluid ejector body containing fluid ejector actuators, ink ejector actuators, and sealant ejector actuators may be inserted into the opening of the enclosing medium wherein the bioactive fluid, the ingestible ink and the sealant are all dispensed in a predetermined sequence depending on the particular application in which the dosage form will be utilized. Another example, is where separate bioactive fluid, ingestible ink, and sealant dispensing stations are utilized, and each dispensing station has a fluid ejector body or bodies dedicated to a particular material.

Optional telescoping insert medium process 984 may be utilized for those applications where it is desirable for either a bioactive agent or multiple agents to be released in a controlled manner. For example, as shown in Fig. 8 multiple inserts may be utilized to provide a predetermined release rate, for either an agent or multiple agents, that may increase, decrease, remain constant, or be pulsed over time. Typically, telescoping insert medium process 984 may be similar to that utilized for loading enclosing medium process 970. In one embodiment, insert medium is loaded or telescoped into the enclosing medium, after the deposition of the bioactive agent, optional ingestible ink and optional sealant material is completed, and before a bioactive agent is dispensed onto the interior surface of the insert medium. In alternate embodiments, separate telescoping and dispensing insert stations may be utilized wherein the desired bioactive fluid is dispensed onto the interior surface of the insert medium before the insert medium is telescoped into the enclosing medium at still another assembly station. In addition, telescoping insert medium process 984 is utilized to position the insert medium to accept insertion of a fluid ejector body into an opening of the insert medium. Typically, this process may utilize similar techniques as that described above for loading enclosing medium process 970.

Optional aligning insert medium process 986 is used to align the opening in the insert medium to the fluid ejector head so that the fluid ejector body may be inserted into the insert medium. Typically, this process may utilize similar techniques as that described above for aligning enclosing medium process 972. Optional inserting insert fluid ejector body process 988 is utilized to insert the insert fluid ejector body into the opening of the insert medium. Typically, this process may utilize similar techniques as that described above for inserting fluid ejector process 974. Optional activating insert fluid ejector actuator process 990 is utilized to eject a bioactive fluid from at least one nozzle disposed on the fluid ejector head. Typically, this process may utilize similar techniques as that described above for activating fluid ejector actuator process 976. Optional dispensing insert bioactive fluid process 992 is utilized to dispense and control the location of the ejected bioactive fluid drops on the inside surface of the insert medium to form the discrete bioactive agent deposits. Typically, this process may utilize similar techniques as that described above for dispensing bioactive fluid process 978 including the rotational and vertical displacement processes.

## Claims

1. A method of making a pharmaceutical dosage form (500, 600, 601, 602, 700), comprising:
inserting (974) a fluid ejector body (120, 220, 320, 420) into an opening (108, 108', 408) of an enclosing medium (106, 106', 406, 506, 606); and
activating (976) a fluid ejector actuator (150, 250, 350, 354, 358) to eject a bioactive fluid onto an interior surface (110, 110', 510, 610, 710) of said enclosing medium as a plurality of discrete deposits (514, 614, 714) on said interior surface, forming an array of bioactive agent particles.

2. The method in accordance with the method of claim 1, further comprises activating (982) a sealant fluid ejector to eject a barrier component fluid (620) over said dispensed bioactive substance.

3. The method in accordance with the method of claim 1, wherein activating said fluid ejector actuator, further comprises activating a fluid energy generating element (252, 352, 356, 360) a predetermined number of times n to eject essentially n drops of said bioactive fluid, in a two-dimensional array, forming an areal density of bioactive agent deposits onto said interior surface of said enclosing medium.

4. The method in accordance with the method of claim 1, further comprising:
inserting (988) an insert fluid ejector body into an opening of an enclosing insert medium (707, 708) having a radial direction;
activating (990) an insert fluid ejector actuator to eject an insert bioactive fluid onto at least a portion of an interior surface (710', 710") of said enclosing insert medium;
dispensing (992) said insert bioactive fluid in an insert two-dimensional array forming an insert areal density of insert bioactive agent deposits onto said enclosing insert medium; and
telescoping (984) said enclosing insert medium into said opening of said enclosing medium, wherein said combination of said discrete deposits on said interior surface and said insert bioactive agent deposits on said enclosing insert medium forms a bioactive agent gradient in said radial direction of said enclosing medium and said enclosing insert medium.

5. The method in accordance with the method of claim 1, wherein activating said fluid ejector actuator further comprises activating a fluid energy generating element (252, 352, 356, 360) depositing essentially a drop of said bioactive fluid onto said interior surface of said enclosing medium for each activation of said fluid energy generating element, wherein said drop is in the range from five femtoliters to ten nanoliters.

6. The method in accordance with the method of claim 1, wherein activating said fluid ejector actuator further comprises activating said fluid ejector actuator to eject a radioactive fluid, said radioactive fluid including a radioactive isotope.

7. A pharmaceutical dosage form (500, 600, 601, 602, 700) comprising:
an enclosing medium (106, 106', 406, 506, 606) having an interior surface (110, 110', 510, 610, 710); **characterized in that** it further comprises
a plurality of discrete deposits (514, 614, 714) of a bioactive substance dispensed on said interior surface (110, 110', 510, 610, 710) of said enclosing medium (106, 106', 406, 506, 606), forming an array of bioactive agent particles.

8. The pharmaceutical dosage form in accordance with claim 7, wherein said enclosing medium is a capsule (508, 706), said capsule having an outer diameter in the range from 3 millimeters to 12 millimeters.

9. The pharmaceutical dosage form in accordance with claim 7, further comprising a sealant material (620) deposited over said plurality of discrete deposits of said bioactive substance.

10. The pharmaceutical dosage form in accordance with claim 7, further comprising at least one insert medium (707, 708) having a two dimensional array of insert discrete deposits of said bioactive substance dispensed on either an interior surface (710', 710") or an outer surface of said at least one insert medium, wherein said at least one insert medium is telescoped into said enclosing medium having a two dimensional array of said discrete deposits of said bioactive substance, wherein said two-dimensional array of discrete deposits of said bioactive substance on said interior surface of said enclosing medium, and said two-dimensional array of insert discrete deposits forms a bioactive substance gradient in a radial direction of said enclosing medium.

11. The pharmaceutical dosage form in accordance with claim 7, wherein said plurality of discrete deposits of said bioactive substance further comprise at least one microparticulate deposit (616) weighing in the range from 1 picogram to 10 micrograms.

12. The pharmaceutical dosage form in accordance with claim 7, wherein said bioactive substance is a radioactive material.

13. A bioactive fluid dispensing system comprising:
at least one bioactive fluid ejector head (100, 100', 200, 300) comprising:
a fluid ejector body (120, 220, 320) adapted to be inserted into an opening (108, 108', 408) of a pharmaceutical enclosing medium (106, 106', 406, 506, 606), said pharmaceutical enclosing medium having an interior surface (110, 110', 510,610,710), **characterized in that** it further comprises
nozzles (130, 230, 330, 332, 334) disposed on said fluid ejector body, and
a fluid ejector actuator (150, 250, 350, 354, 358) in fluid communication with said nozzles,
a fluid controller (490) operably coupled to said fluid ejector actuator, and
at least one enclosing medium holder (486) adapted to hold said pharmaceutical enclosing medium, wherein said at least one fluid ejector head and said at least one enclosing medium holder are arranged such that, when said at least one enclosing medium holder (486) is holding said pharmaceutical enclosing medium, activation of said fluid ejector actuator ejects a bioactive fluid through said nozzles onto predetermined locations on said interior surface of said pharmaceutical enclosing medium, forming an array of bioactive agent particles.

14. The fluid dispensing system in accordance with claim 13, wherein, when said at least one enclosing medium holder (486) is holding said pharmaceutical enclosing medium, activation of said fluid ejector actuator ejects said bioactive fluid in a two-dimensional array onto said interior surface of said pharmaceutical enclosing medium.

## Patentansprüche

1. Ein Verfahren zur Herstellung einer pharmazeutischen Dosierungsform (500, 600, 601, 602, 700), das folgende Schritte aufweist:
Einbringen (974) eines Fluid-Auswerfer-Körpers (120, 220, 320, 420) in eine Öffnung (108, 108', 408) eines umschließenden Mediums (106, 106', 406, 506, 606); und
Aktivieren (976) eines Fluid-Auswerfer-Betätigungselements (150, 250, 350, 354, 358) zum Auswerfen eines bioaktiven Fluids auf eine innere Oberfläche (110, 110', 510, 610, 710) des umschließenden Mediums als eine Mehrzahl von einzelnen Ablagerungen (514, 614, 714) auf der inneren Oberfläche, wobei ein Array von Partikeln eines bioaktiven Agens gebildet wird.

2. Das Verfahren gemäß dem Verfahren von Anspruch 1, das ferner ein Aktivieren (982) eines Dichtmittel-Fluid-Auswerfers umfasst, zum Auswerfen eines Barriere-Komponenten-Fluids (620) über die abgegebene bioaktive Substanz.

3. Das Verfahren gemäß dem Verfahren von Anspruch 1, bei dem das Aktivieren des Fluid-Auswerfer-Betätigungselements ferner ein Aktivieren eines Fluid-Energie-Erzeugungs-Elements (252, 352, 356, 360) zu einer vorbestimmten Anzahl von Malen n umfasst, zum Auswerfen von im Wesentlichen n Tropfen des bioaktiven Fluids in einem zweidimensionalen Array, wobei eine Flächendichte von Ablagerungen eines bioaktiven Agens auf der inneren Oberfläche des umschließenden Mediums gebildet wird.

4. Das Verfahren gemäß dem Verfahren von Anspruch 1, das ferner folgende Schritte aufweist:
Einbringen (988) eines Einbringungs-Fluid-Auswerfer-Körpers in eine Öffnung eines umschließenden Einbringungsmediums (707, 708) mit einer Radialrichtung;
Aktivieren (990) eines Einbringungs-Fluid-Auswerfer-Betätigungselements, zum Auswerfen eines bioaktiven Einbringungs-Fluids auf zumindest einen Abschnitt einer inneren Oberfläche (710', 710") des umschließenden Einbringungsmediums;
Abgeben (992) des bioaktiven Einbringungs-Fluids in einem zweidimensionalen Einbringungs-Array, das eine Einbringungs-Flächendichte von Einbringungs-Ablagerungen eines bioaktiven Agens bildet auf dem umschließenden Einbringungsmedium; und
Ineinanderschieben (984) des umschließenden Einbringungsmediums in die Öffnung des umschließenden Mediums, wobei die Kombination der einzelnen Ablagerungen auf der inneren Oberfläche und der Einbringungs-Ablagerungen des bioaktiven Agens auf dem umschließenden Einbringungsmedium einen Bioaktives-Agens-Gradienten bildet in der Radialrichtung des umschließenden Mediums und des umschließenden Einbringungsmediums.

5. Das Verfahren gemäß dem Verfahren von Anspruch 1, bei dem das Aktivieren des Fluid-Auswerfer-Betätigungselements ferner ein Aktivieren eines Fluid-Energie-Erzeugungs-Elements (252, 352, 356, 360) umfasst, das im Wesentlichen einen Tropfen des bioaktiven Fluids auf die innere Oberfläche des umschließenden Mediums aufbringt für jede Aktivierung des Fluid-Energie-Erzeugungs-Elements, wobei der Tropfen im Bereich von fünf Femtolitern bis zehn Nanolitern liegt.

6. Das Verfahren gemäß dem Verfahren von Anspruch 1, bei dem das Aktivieren des Fluid-Auswerfer-Betätigungselements ferner ein Aktivieren des Fluid-Auswerfer-Betätigungselements umfasst, zum Auswerfen eines radioaktiven Fluids, wobei das radioaktive Fluid ein radioaktives Isotop umfasst.

7. Eine pharmazeutische Dosierungsform (500, 600, 601, 602, 700), die folgende Merkmale aufweist:
ein umschließendes Medium (106, 106', 406, 506, 606), das eine innere Oberfläche (110, 110', 510, 610, 710) aufweist, **gekennzeichnet dadurch, dass** dieselbe ferner folgende Merkmale aufweist:
eine Mehrzahl von einzelnen Ablagerungen (514, 614, 714) einer bioaktiven Substanz, abgegeben auf die innere Oberfläche (110, 110', 510, 610, 710) des umschließenden Mediums (106, 106', 406, 506, 606), wobei ein Array von Partikeln eines bioaktiven Agens gebildet wird.

8. Die pharmazeutische Dosierungsform gemäß Anspruch 7, bei der das umschließende Medium eine Kapsel (508, 706) ist, wobei die Kapsel einen Außendurchmesser im Bereich von 3 Millimetern bis 12 Millimetern aufweist.

9. Die pharmazeutische Dosierungsform gemäß Anspruch 7, die ferner ein Dichtmittelmaterial (620) aufweist, aufgebracht über die Mehrzahl von einzelnen Ablagerungen der bioaktiven Substanz.

10. Die pharmazeutische Dosierungsform gemäß Anspruch 7, die ferner zumindest ein Einbringungsmedium (707, 708) aufweist, das ein zweidimensionales Array von einzelnen Einbringungs-Ablagerungen der bioaktiven Substanz aufweist, abgegeben entweder auf eine innere Oberfläche (710', 710") oder eine äußere Oberfläche des zumindest einen Einbringungsmediums, wobei das zumindest eine Einbringungsmedium in das umschließende Medium eingeschoben ist, das ein zweidimensionales Array der einzelnen Ablagerungen der bioaktiven Substanz aufweist, wobei das zweidimensionale Array von einzelnen Ablagerungen der bioaktiven Substanz auf der inneren Oberfläche des umschließenden Mediums und das zweidimensionale Array von einzelnen Einbringungs-Ablagerungen einen Bioaktive-Substanz-Gradienten in einer Radialrichtung des umschließenden Mediums bilden.

11. Die pharmazeutische Dosierungsform gemäß Anspruch 7, bei der die Mehrzahl von einzelnen Ablagerungen der bioaktiven Substanz ferner zumindest eine Mikropartikel-Ablagerung (616) aufweist, die im Bereich von 1 Picogramm bis 10 Mikrogramm wiegt.

12. Die pharmazeutische Dosierungsform gemäß Anspruch 7, bei der die bioaktive Substanz ein radioaktives Material ist.

13. Ein Bioaktives-Fluid-Abgabe-System, das folgende Merkmale aufweist:
zumindest einen Bioaktives-Fluid-Auswerfer-Kopf (100, 100', 200, 300), der folgende Merkmale aufweist:
einen Fluid-Auswerfer-Körper (120, 220, 320), angepasst, um in eine Öffnung (108, 108', 408) eines pharmazeutischen umschließenden Mediums (106, 106', 406, 506, 606) eingebracht zu sein; wobei das pharmazeutische umschließende Medium eine innere Oberfläche (110, 110', 510, 610, 710) aufweist, **gekennzeichnet dadurch, dass** derselbe ferner folgende Merkmale aufweist:
Düsen (130, 230, 330, 332, 334), angeordnet auf dem Fluid-Auswerfer-Körper, und
ein Fluid-Auswerfer-Betätigungselement (150, 250, 350, 354, 358) in Fluidkommunikation mit den Düsen,
eine Fluid-Steuerung (490), wirksam gekoppelt an das Fluid-Auswerfer-Betätigungselement; und
zumindest eine Umschließendes-Medium-Haltevorrichtung (486), angepasst, um das pharmazeutische umschließende Medium zu halten, wobei der zumindest eine Fluid-Auswerfer-Kopf und die zumindest eine Umschließendes-Medium-Haltevorrichtung so angeordnet sind, dass, wenn die zumindest eine Umschließendes-Medium-Haltevorrichtung (486) das pharmazeutische umschließende Medium hält, eine Aktivierung des Fluid-Auswerfer-Betätigungselements ein bioaktives Fluid durch die Düsen auf vorbestimmte Stellen auf der inneren Oberfläche des pharmazeutischen umschließenden Mediums auswirft, wobei ein Array von Partikeln eines bioaktiven Agens gebildet wird.

14. Das Fluid-Abgabe-System gemäß Anspruch 13, bei dem, wenn die zumindest eine Umschließendes-Medium-Haltevorrichtung (486) das pharmazeutische umschließende Medium hält, eine Aktivierung des Fluid-Auswerfer-Betätigungselements das bioaktive Fluid in einem zweidimensionalen Array auf die innere Oberfläche des pharmazeutischen umschließenden Mediums auswirft.

## Revendications

1. Procédé de réalisation d'une forme posologique pharmaceutique (500, 600, 601, 602, 700), comprenant :
l'insertion (974) d'un corps éjecteur de fluide (120, 220, 320, 420) dans une ouverture (108, 108', 408) d'un support d'enveloppement (106, 106', 406, 506, 606) ; et
l'activation (976) d'un actionneur éjecteur de fluide (150, 250, 350, 354, 358) pour éjecter un fluide bioactif sur une surface intérieure (110, 110', 510, 610, 710) dudit support d'enveloppement comme une pluralité de dépôts discrets (514, 614, 714) sur ladite surface intérieure, formant un réseau de particules d'agent bioactif.

2. Procédé en conformité avec le procédé de la revendication 1, qui comprend en outre l'activation (982) d'un éjecteur de fluide de scellement pour éjecter un fluide formant composant barrière (620) sur ladite substance distribuée.

3. Procédé en conformité avec le procédé de la revendication 1, dans lequel l'activation dudit actionneur éjecteur de fluide, comprend en outre l'activation d'un élément de génération d'énergie de fluide (252, 352, 356, 360) un nombre prédéterminé de fois n pour éjecter essentiellement n gouttelettes dudit fluide bioactif, dans un réseau en deux dimensions, formant une densité de surface de dépôts d'agent bioactif sur ladite surface intérieur dudit support d'enveloppement.

4. Procédé en conformité avec le procédé de la revendication 1, comprenant en outre :
l'insertion (988) d'un corps éjecteur de fluide d'insert dans une ouverture d'un support d'insert d'enveloppement (707, 708) ayant une direction radiale ;
l'activation (990) d'un actionneur éjecteur de fluide d'insert pour éjecter un fluide bioactif d'insert sur au moins une partie d'une surface intérieure (710', 710'') dudit support d'insert d'enveloppement ;
la distribution (992) dudit fluide bioactif d'insert dans un réseau en deux dimensions d'insert, formant une densité de surface d'insert de dépôts d'agent bioactif d'insert sur ledit support d'insert d'enveloppement ; et
l'enroulage télescopique (984) dudit support d'insert d'enveloppement dans ladite ouverture dudit support d'enveloppement, dans lequel ladite combinaison desdits dépôts discrets sur ladite surface intérieure et desdits dépôts d'agent bioactif d'insert sur ledit support d'insert d'enveloppement forme un gradient d'agent bioactif dans ladite direction radiale dudit support d'enveloppement et dudit support d'insert d'enveloppement.

5. Procédé en conformité avec le procédé de la revendication 1, dans lequel l'activation dudit actionneur éjecteur de fluide comprend en outre l'activation d'un élément de génération d'énergie de fluide (252, 352, 356, 360) déposant essentiellement une goutte dudit fluide bioactif sur ladite surface intérieure dudit support d'enveloppement pour chaque activation dudit élément de génération d'énergie de fluide, dans lequel ladite goutte est dans la plage de cinq femtolitres à dix nano litres.

6. Procédé en conformité avec le procédé de la revendication 1, dans lequel l'activation dudit actionneur éjecteur de fluide comprend en outre l'activation dudit actionneur d'éjecteur de fluide pour éjecter un fluide radioactif, ledit fluide radioactif comprend un isotope radioactif.

7. Forme posologique pharmaceutique (500, 600, 601, 602, 700) comprenant :
un support d'enveloppement (106, 106', 406, 506, 606) ayant une surface intérieure (110, 110', 510, 610, 710) ; **caractérisée en ce qu'**elle comprend en outre
une pluralité de dépôts discrets (514, 614, 714) d'une substance bioactive distribuée sur ladite surface intérieure (110, 110', 510, 610, 710) dudit support d'enveloppement (106, 106', 406, 506, 606), formant un réseau de particules d'agent bioactif.

8. Forme posologique pharmaceutique selon la revendication 7, dans laquelle ledit support d'enveloppement est une capsule (508, 706), ladite capsule ayant un diamètre externe dans la plage de 3 millimètres à 12 millimètres.

9. Forme posologique pharmaceutique selon la revendication 7, comprenant en outre un matériau de scellement (620) déposé sur ladite pluralité de dépôts discrets de ladite substance bioactive.

10. Forme posologique pharmaceutique selon la revendication 7, comprenant en outre au moins un support d'insert (707, 708) ayant un réseau en deux dimensions de dépôts discrets d'insert de ladite substance bioactive distribuée sur une surface intérieure (710', 710'') ou une surface externe dudit au moins un support d'insert, dans lequel ledit au moins un support d'insert est enroulé de manière télescopique dans ledit support d'enveloppement ayant un réseau en deux dimensions desdits dépôts discrets de ladite substance bioactive, dans laquelle ledit réseau en deux dimensions de dépôts discrets de ladite substance bioactive sur ladite surface intérieure dudit support d'enveloppement, et ledit réseau en deux dimensions de dépôts discrets d'insert forme un gradient de substance bioactive dans une direction radiale dudit support d'enveloppement.

11. Forme posologique pharmaceutique selon la revendication 7, dans laquelle ladite pluralité de dépôts discrets de ladite substance bioactive comprend en outre au moins un dépôt micro particulaire (616) ayant un poids de l'ordre de 1 pico gramme à 10 microgrammes.

12. Forme posologique pharmaceutique selon la revendication 7, dans laquelle ladite substance bioactive est un matériau radioactif.

13. Système de distribution de fluide bioactif comprenant :
au moins une tête d'éjecteur de fluide bioactif (100, 100', 200, 300) comprenant :
un corps d'éjecteur de fluide (120, 220, 320) adapté à être inséré dans une ouverture (108, 108', 408) d'un support d'enveloppement pharmaceutique (106, 106', 406, 506, 606), ledit support d'enveloppement pharmaceutique ayant une surface intérieure (110, 110', 510, 610, 710),
**caractérisé en ce qu'**il comprend en outre des buses (130, 230, 330, 332, 334) disposées sur ledit corps éjecteur de fluide, et
un actionneur éjecteur de fluide (150, 250, 350, 354, 358) en communication de fluide avec lesdites buses,
un contrôleur de fluide (490) couplé de manière opérationnelle audit actionneur éjecteur de fluide ; et
au moins un élément de maintien de support d'enveloppement (486) adapté pour maintenir ledit support d'enveloppement pharmaceutique, dans lequel ladite au moins une tête d'éjecteur de fluide et ledit au moins un élément de maintien de support d'enveloppement sont agencés de telle sorte que, lorsque ledit au moins un élément de maintien de support d'enveloppement (486) maintient ledit support d'enveloppement pharmaceutique, l'activation dudit actionneur éjecteur de fluide éjecte un fluide bioactif à travers lesdites buses sur des emplacements prédéterminés sur ladite surface intérieure dudit support d'enveloppement pharmaceutique, formant un réseau de particules d'agent bioactif.

14. Système de distribution de fluide selon la revendication 13, dans lequel, lorsque ledit au moins un élément de maintien de support d'enveloppement (486) maintient ledit support d'enveloppement pharmaceutique, l'activation dudit actionneur éjecteur de fluide éjecte ledit fluide bioactif dans un réseau en deux dimensions sur ladite surface intérieure dudit support d'enveloppement pharmaceutique.
